# EUROPÄISCHE PATENTSCHRIFT

(11) **EP 1 095 007 B1**
(45) Veröffentlichungstag und Bekanntmachung des Hinweises auf die Patenterteilung: **04.06.2003**
(21) Anmeldenummer: 99934636.4
(22) Anmeldetag: 08.07.1999
(51) Int. Cl.: C07C 59/19, C07C 51/41, A61K 31/19

(54) **WASSERLÖSLISCHE ZINKPYRUVATE BZW. DEREN HYDRATE, VERFAHREN ZU IHRER HERSTELLUNG UND DEREN VERWENDUNG**
WATER-SOLUBLE ZINC PYRUVATES OR THEIR HYDRATES, METHOD FOR THE PRODUCTION THEREOF AND THEIR USE
PYRUVATES DE ZINC SOLUBLES DANS D'EAU ET LEURS HYDRATES, PROCEDE PERMETTANT DE LES PREPARER ET LEUR UTILISATION

(30) Priorität: 09.07.1998 DE 19830770
(43) Veröffentlichungstag der Anmeldung: 02.05.2001
(73) Patentinhaber: Finzelberg GmbH & Co. KG, 56626 Andernach (DE)
(72) Erfinder: PISCHEL, Ivo, D-83342 Tacherting (DE); PARADIES, Henrich, Hasko, D-58644 Iserlohn (DE)
(74) Vertreter: Weiss, Wolfgang, Dipl.-Chem. Dr.
(86) Internationale Anmeldenummer: EP9904812
(87) Internationale Veröffentlichungsnummer: WO00002841

(56) Entgegenhaltungen:
- WO-A-92/15292
- G. WEITZEL AL AL: HOPPE-SEYLERS Z. PHYSIOL. CHEM., Nr. 292, 1953, Seiten 286-302, XP002116797
- J.J. BERZELIUS: ANNALEN DER PHYSIK UND CHEMIE, Bd. 36, Nr. 9, 1835, Seiten 1-29, XP002116798 in der Anmeldung erwähnt
- G. FISCHER ET AL: J. ORG. CHEM., Bd. 53, Nr. 1, 1988, Seiten 214-216, XP002116874

## Beschreibung

Die vorliegende Erfindung betrifft wasserlösliche Zinkpyruvate bzw. deren Hydrate mit einer hohen Reinheit und einer guten Lager- und Thermostabilität, ein Verfahren zu ihrer Herstellung und deren Verwendung.

Es ist bekannt, daß Salze der Brenztraubensäure, die als Pyruvate bezeichnet werden, wertvolle physiologische, therapeutische und diätetische Eigenschaften besitzen. Pyruvate finden Anwendung zur Steigerung der Ausdauer und Kraft im Sportbereich, zur Gewichts- und Körperfettreduzierung sowie als Schutzsubstanz für Körperzellen und - gewebe, besonders kardiovaskuläres, hepatisches, nephrotisches, peritoneales und neuronales Gewebe, und als Antioxidans, sowohl als Substanz zur Inhibition der Radikalbildung als auch als Radikalfängersubstanz in Körperzellen, -geweben und Zellen des Synovialgewebes, im Gesundheitsbereich und als Nahrungsergänzungsmittel. Des weiteren finden Pyruvate als Wundheilmittel, zur Diabetesbehandlung wegen ihrer blutzuckersenkenden Wirkung und zur Behandlung von Nierenerkrankungen (akutes Nierenversagen, Nierensteinleiden) Anwendung.

Auf der anderen Seite ist seit über 100 Jahren bekannt, daß Zink ein essentielles Spurenelement für Pflanzen, Tiere und Menschen darstellt. Das Auftreten eines Zinkmangels wurde beim Menschen lange Zeit für unwahrscheinlich gehalten. Folglich war daher zunächst das Interesse der Ernährungswissenschaft an diesem Spurenelement gering. In den 50er Jahren wurde dann der Nachweis erbracht, daß beim Menschen ein Zinkmangel mit typischen Symptomen auftreten kann. Die Forschung wurde weltweit intensiviert, als 1961 das endemische Auftreten von Hypogonadismus und Zwergenwuchs im Iran und im Nildelta Ägyptens auf einen Zinkmangel zurückgeführt werden konnte. Analytische Routinemessungen in Lebensmitteln und Gewebeproben trugen zum besseren Verständnis der Rolle des Spurenelements Zink in biochemischen Prozessen bei. Heute sind die klinischen Symptome eines schweren Zinkmangels bekannt, die im Zinkmangel auftretenden pathologischen Veränderungen können aber bisher nicht erklärt werden.

Zink ist Bestandteil von Metalloenzymen und stabilisiert organische Strukturen sowie Membranen. Inzwischen konnten über 200 verschiedene zinkabhängige Enzyme identifiziert werden. Die Superoxiddismutase, die alkalische Phosphatase, die DNS- und RNS-Polymerasen und die Carboxypeptidase sind Beispiele dafür. Zink ist an der Nukleinsäuresynthese, dem Protein-, Lipid- und Kohlenhydratstoffwechsel, dem Knochenstoffwechsel, dem Sauerstofftransport, der Dunkeladaptation und dem antioxidativen Schutzsystems unseres Organismus beteiligt. Zink ist für alle Lebensformen essentiell, weil es eine wichtige Rolle bei der Transkription und Translation sowie der Expression von Genen spielt. Einige Enzyme enthalten Zink im aktiven Zentrum, wobei es dort als Elektronenakzeptorfungiert. In anderen Enzymen und Nichtenzym-Proteinen sowie in Nukleinsäuren hat Zink strukturelle und stabilisierende Funktionen (Zinkfinger). Im Pankreas ist Zink an der Insulinsynthese und -speicherung beteiligt. Des weiteren erfüllt es wichtige Aufgaben im Immunsystem.

Bei Zink ist neben dem absoluten Gehalt auch die Bioverfügbarkeit entscheidend für die Bedeutung eines Lebensmittels als Zinkquelle. Die Absorption von Zink aus Lebensmitteln tierischen Ursprungs ist im allgemeinen höher als aus Lebensmitteln pflanzlicher Herkunft. Zink, das mit Phytinsäure schlecht absorbierbare Komplexe bildet, ist aus phytatreichen Lebensmitteln (z. B. Vollkorngetreide) schlecht verfügbar. Ebenso absorptionshemmend wirken gleichzeitig erhöhte Aufnahmen der Metalle Eisen, Kupfer, Zinn und Cadmium. Die Anwesenheit von Aminosäuren, Peptiden und organischen Säuren erhöht die Bioverfügbarkeit. Muskelfleisch, Milchprodukte, Fisch und besonders Schalentiere (z.B. Austern bis zu 160 mg Zn/100 g) sind gute Zinkquellen. Dunkles Fleisch (Rind: 4,3 mg Zn/100 g) hat einen höheren Zinkgehalt als helles Fleisch (Huhn: 1,0 mg Zn/100 g). Die meisten Obst- und Gemüsesorten tragen nur wenig zur Zinkversorgung bei. Der Zinkgehalt von Getreideprodukten ist stark abhängig vom Verarbeitungsgrad, da Zink überwiegend in den Randschichten des Korns lokalisiert ist. Die durchschnittliche Absorptionsrate von Zink aus verschiedenen Brotsorten beträgt rund 10 %, wohingegen diese aus Fleisch unterschiedlicher Geflügelarten bis zu 40 % betragen kann.

Das Zink wird durch Verdauungsenzyme und Magensäure aus der Lebensmittelbindung teilweise freigesetzt, und bindet sich locker an niedrigmolekulare Liganden wie Aminosäuren, Peptide, organische Säuren und Phosphate, was von Bedeutung für den Umfang der Zinkabsorption ist. Zink wird durch einen sättigbaren Carrier-Prozeß im Duodenum und Jejunum absorbiert. Absorbiertes Zink wird im Blutplasma an Albumine, a₂-Makroglobulin und Transferrin gebunden und transportiert. Der an Plasmaproteine gebundene Zinkanteil macht allerdings nur ca. 20 % des Zinkgehalts im Blut aus. Der größte Anteil (75 %) liegt als Bestandteil der Carboanhydratase in den Erythrozyten vor. Weitere 3 % befinden sich als Bestandteil der alkalischen Phosphatase in den Leukozyten.

Zink findet sich in unterschiedlichen Konzentrationen in allen Körpergeweben und -flüssigkeiten. Der Körperbestand des Erwachsenen beträgt insgesamt 2 - 3 g Zink. Ca. 60 % liegen in der Skelettmuskulatur und 30 % im Knochen vor. Zink ist in erster Linie ein intrazelluläres Ion, das in extrazellulären Flüssigkeiten nur in relativ geringen Konzentrationen vorkommt. Im Plasma zirkulieren nur 0,1 % des Gesamtkörperzinks. Dort unterliegt der Zinkgehalt einer strengen homöostatischen Kontrolle. Der Mensch ist auf eine regelmäßige Zinkaufnahme mit der Nahrung angewiesen. Zink wird überwiegend durch den Darm ausgeschieden. Es geht hier mit Verdauungssekreten und abgeschuppten Darmepithelzellen verloren. Die endogenen Verluste können 2 bis 4 mg/Tag betragen. Jeweils weitere 0,5 mg werden mit dem Urin und über die Haut (Hautabschilferungen, Haare und Schweiß) ausgeschieden. Bei entzündlichen Darm- und Nierenerkrankungen sowie bei Alkoholismus ist die Ausscheidung signifikant erhöht.

Schwere Formen eines Zinkmangels wurden bei Menschen mit angeborenen Störungen des Zinkstoffwechsels (Acrodermatitis enteropathica), bei Patienten mit unzulänglicher parenteraler Ernährung und bei Morbus-Crohn-Patienten beobachtet. Das klinische Erscheinungsbild umfaßt eine Dermatitis an den Enden der Gliedmaßen und um den Mund, eine Diarrhoe, Appetitlosigkeit, Haarausfall und neuropsychiatrische Störungen, Gedeihund Wachstumsdepressionen, eine erhöhte Infektanfälligkeit sowie eine verzögerte Wundheilung und eine Störung der sexuellen Entwicklung.

Des weiteren ist Zink zur Prävention der progredienten Verschlechterung der Glukosetoleranz im Alter anwendbar. Mit zunehmendem Alter kommt es unabhängig vom Körpergewicht zu einer hochsignifikanten Verschlechterung der Glukosetoleranz und zu einer signifikanten Verminderung der Insulinsekretion nach Glukosegabe. Die basalen Serum-Insulin-Werte nehmen mit steigendem Alter ab. Für den Glukosestoffwechsel des Gehirns konnte eine Reduktion der Glukoseumsatzraten mit der Alterung nachgewiesen werden. Tierexperimentelle und klinische Untersuchungsergebnisse zeigen, daß Mangel an Zink bei der progredienten Verschlechterung der Glukosetoleranz im Alter eine integrierende Rolle spielt.

Besonders eignet sich Zink zur Behandlung von Diabetes mellitus. Zink verbessert die Glukosetoleranz und kann die Insulinwirkung deutlich erhöhen. Zink ist ein bedeutendes Therapeutikum für Diabetiker. In Deutschland leben nach Angaben der Deutschen Diabetes-Gesellschaft (DDG) gegenwärtig circa 4 Mio. Diabetiker (5 % Typ-I-Diabetiker, 95 % Typ-II-Diabetiker>. Nach Schätzung führender Diabetologen wird sich diese Zahl bis zum Jahr 2000 verdoppeln.

Die Hyperglykämie kennzeichnetals Kardinalsymptom den Diabetes mellitus. Bei Diabetikern liegen entweder ein Insulinmangel und/oder eine Insulinresistenz vor. Insulin reguliert den Blutglucosespiegel. Spurenelemente können keinen Diabetiker heilen oder allein therapieren. Trotzdem spielt das lebensnotwendige Spurenelement Zink eine entscheidende Rolle bei der Blutzuckerregulation. Bei Zinkmangel kommt es gehäuft zu pathologischer Glucosetoleranz, die alleine durch regelmäßige Zinkgabe normalisiert werden kann.

Das lebensnotwendige Spurenelement Zink verbessert die Insulinspeicherung bei Typ-II-Diabetikern. Zinkmangel hemmt die Insulinaktivität und die Insulinrezeptorbildung. Der tägliche Zinkbedarf beträgt laut der Deutschen Gesellschaft für Ernährung (DGE) im Erwachsenenalter 12 bis 15 Milligramm. Die durchschnittliche Zinkaufnahme über Nahrungsmittel (besonders in Austern, Innereien, Fleisch) liegt etwas unter dieser Empfehlung. Diabetiker gehören zu den Risikogruppen der Zinkversorgung, da deren Urin-Zink-Verluste deutlich gesteigert sind. Die Höhe der Zinkausscheidung steigt mit der Ausprägung der Glucosurie (Harnzuckerausscheidung). Dies trifft insbesondere auf Diabetiker zu, die unter diabetischer Nephropathie leiden. Die McNair-Studie zeigt Urin-Zink-Ausscheidungen, die bei Diabetikern im Vergleich zu Gesunden um 50 bis 150 % gesteigert sind. Bei positiven Harnzuckerwerten ist der Zinkverlust noch stärker und kann das 2- bis 3fache der Norm überschreiten. Bei Typ-II-Diabetikern fanden Perger und Mitarbeiter nach 6 Wochen Zinkgabe eine Senkung der durchschnittlichen Nüchtern-Blutzucker-Werte von 250 auf 142 mg %. In anderen Studien fällt sowohl der Nüchtern-, als auch der postprandiale Blutzucker-Spiegel unter Zinkgabe deutlich ab. Daher scheint die Einnahme von 15 bis 30 Milligramm Zink täglich über Tabletten sinnvoll.

Unter Zinkgabe heilen Wunden besser ab, z.B. bei dem sogenannten Diabetischen Fuß auf dem Boden von schlechten Blutzuckerwerten, peripherer Verschlußkrankheit und diabetischer Polyneuropathie. Dies ist u. a. auf den entzündungshemmenden Effekt von Zink zurückzuführen.

Der genaue Zinkbedarf des Menschen ist unbekannt. Nach Bilanzstudien betragen die täglich zu ersetzenden, obligatorischen Zinkverluste 2,5 mg. Bei einer durchschnittlichen Bioverfügbarkeit von 20 % wurde hieraus in Deutschland eine empfohlene Tageszufuhr von 15 mg für Männer abgeleitet. Wegen des geringeren durchschnittlichen Körpergewichts wird für Frauen eine Zinkaufnahme von 12 mg/Tag empfohlen. In den USA beträgt die empfohlene Tagesdosis (RDI) für Erwachsene 15 mg Zink.

In der Literatur ist Zinkpyruvat von J.J. Berzelius, Annalen der Physik und Chemie (1835) 36, 20 als Zinkoxydsalz der brenzlichen Traubensäure beschrieben. Im Beilstein, Band 3 des Hauptwerkes, Seite 612, ist diese Literaturstelle zitiert unter der Angabe des Zinkpyruvat als Trihydrat.

Berzelius gewann das Salz entweder durch Umsetzung von basischem Zinkcarbonat oder von metallischem Zink mit verdünnter Brenztraubensäure. Das erhaltene Salz soll nach Berzelius ein schwerlösliches Pulver sein. Die Nacharbeitung der Herstellungsvorschrift von Zinkpyruvat nach Berzelius ergibt jedoch ein schwerlösliches Pulver, das aufgrund moderner analytischer Untersuchungsmethoden (NMR, IR, HPLC) eindeutig als Zinkparapyruvat identifiziert werden konnte.

Es hat sich außerdem gezeigt, daß das bisher bekannte Verfahren zur Herstellung von sog. "Zinkpyruvaten" durch Neutralisation von Brenztraubensäure mit Zinkcarbonat, -hydroxid oder -oxid sowie die Reaktion von Zinkmetall mit Brenztraubensäure zu einem schwerlöslichen Zinkparapyruvat mit großen Mengen an Nebenprodukten führt, da die Brenztraubensäure bzw. die Pyruvationen durch Aldoladditions-oder Aldolkondensationsreaktionen zu acyclischen oder cyclischen Dimeren und Polymeren der Brenztraubensäure reagieren.

Derartig verunreinigte Zinkparapyruvate sind für die Anwendung als Therapeutikum nicht geeignet und des weiteren nicht ausreichend lagerstabil, weil bei der Lagerung unter anderem dimere, polymere und cyclische Verbindungen gebildet werden, die toxisch sein können.

Um Zinkparapyruvate handelt es sich auch bei den als "Zinkpyruvaten" klassifizierten Verbindungen, deren Einfluß auf den Blutzucker von Weitzel et al. untersucht wurde (G. Weitzel et al., Hoppe-Seylers Z. Physiol. Chemie, Nr. 292, 1953, 286-302). Die als "Zinkpyruvat" bezeichnete Verbindung war so schwerlöslich, dass mit ihr keine verwertbare Beeinflussung des Blutzuckers bei Kaninchen erzielt werden konnte (s. S. 290 der Publikation).

Als acyclische Verbindungen sind die Parabrenztraubensäure (4-Hydroxy-4-methyl-2-oxoglutarsäure) und ihre Salze sowie die höheren Aldoladditionsprodukte zu nennen. Des weiteren können Oxal- und Methylbernsteinsäure als Nebenprodukte gebildet werden. Aus den acyclischen Brenztraubensäurepolymeren können durch Lactonisieruhgs-, Ketalisierungs- und andere Reaktionen cyclische Verbindungen entstehen, wie z.B. 2-Hydroxy-2-methyl-4-oxoglutarsäure-5-lacton, Trimesinsäure-, Isophthalsäure- und Pyrantricarbonsäurederivate (Lit.: Beilstein, Hauptwerk Bd. 3, S. 608 - 613; 1. Ergänzungswerk, S.217 - 219; 2. Ergänzungswerk, S.393 - 401; 3. Ergänzungswerk, S.1146 - 1156; 4. Ergänzungswerk, S.1505 - 1510). Diese Nebenprodukte treten in gleicher Weise bei der Lagerung von Zinkpyruvaten auf, die nach dem bisher bekannten Verfahren hergestellt werden. Diese vorangenannten acyclischen und cyclischen Neben- und Zersetzungsprodukte der Brenztraubensäure und ihrer Salze können physiologisch unverträglich oder toxisch sein.

Der vorliegenden Erfindung lag daher die Aufgabe zugrunde, wasserlösliche und lagerstabile Zinkpyruvate zu entwickeln, welche die genannten Nachteile entsprechend dem Stand der Technik nicht aufweisen, sondern eine hohe Reinheit aufweisen und deshalb physiologisch unbedenklich sind.

Diese Aufgabe wurde erfindungsgemäß durch die Bereitstellung der Zinkpyruvate bzw. deren Hydrate der Formel 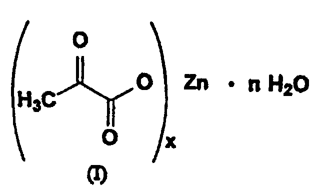 mit x = 1,8 bis 2,2 und n = 0 bis 5 gelöst.

Es hat sich nämlich überraschenderweise gezeigt, daß die erfindungsgemäßen Zinkpyruvate eine hohe Reinheit besitzen und praktisch frei von irgendwelchen toxischen Nebenprodukten sind sowie eine ausgezeichnete Lager- und Thermostabilität aufweisen.

Dies war deshalb so überraschend, weil bei der erfindungsgemäßen Herstellung die Brenztraubensäure keine oder nur in sehr geringem Umfang Kondensations- und Zersetzungsreaktionen unter Bildung von Aldoladdukten eingeht, obwohl Übergangsmetalle wie Zink die Polymerisation von Brenztraubensäure katalysieren sollten.

Die erfindungsgemäßen Zinkpyruvate, die eine gute Wasserlöslichkeit besitzen und durch Elementaranalyse, IR-, NMR-Spektroskopie sowie durch HPLC-Gehaltsbestimmung eindeutig charakterisierbar sind, enthalten das Zinkkation und das Pyruvatanion, welches als Oxopropionat- und/oder als 2,2-Dihydroxypropionat-Anion vorliegen kann, im Molverhältnis 1,8 bis 2,2 : 1 und vorzugsweise im stöchiometrischen Verhältnis von 2 : 1.

Außerdem können die erfindungsgemäßen Zinkpyruvate entweder völlig wasserfrei oder in Form der Hydrate vorliegen, wobei n = O bis 5 und vorzugsweise 0 bis 3 sein kann. Gemäß einer bevorzugten Ausführungsform liegen die Zinkpyruvate in einer festen mikrokristallinen Form vor, die sich durch eine besonders gute Lagerstabilität auszeichnet.

Der Gehalt an Zinkpyruvat kann somit 72,7 bis 100 % und der Kristallwassergehalt von 0 bis 27,3 % (entsprechend 0 bis 5 Mol Kristallwasser) betragen. Bezogen auf die wasserfreie Substanz beträgt der Gehalt an Zinkpyruvat 99 bis 100 %.

Die Herstellung der erfindungsgemäßen Zinkpyruvate erfolgt durch Umsetzung von Zinksalzen organischer Säuren oder acider organischer Keto-oder Hydroxyverbindungen mit Brenztraubensäure im Temperaturbereich von - 20 bis + 90 °C, ggf. in Gegenwart eines Löse- oder Verdünnungsmittels. Vorzugsweise wird die Umsetzung bei 10 bis 50 °C durchgeführt.

Als organische Säure kann im Prinzip jede physiologisch unbedenkliche Carbonsäure eingesetzt werden, die ggf. noch mit Amino-, Keto- oder Hydroxygruppen substituiert sein kann. Vorzugsweise setzt man als organische Säure oder acide organische Keto- oder Hydroxyverbindung eine Säure ausgewählt aus der Gruppe Ameisensäure, Essigsäure, Propionsäure, Buttersäure, Milchsäure, Ascorbinsäure, Citronensäure, Weinsäure, Bernsteinsäure, Maleinsäure, Fumarsäure, Äpfelsäure, Asparginsäure, Benzoesäure, Gluconsäure, Isovaleriansäure, Ölsäure, Glycin oder Lysin sein.

Diese Zinksalze der organischen Säuren oder aciden organischen Keto-oder Hydroxyverbindungen können in wasserfreier Form, als Hydrate oder als feuchte Produkte verwendet werden.

Auch die Brenztraubensäure kann beim erfindungsgemäß vorgeschlagenen Verfahren wahlweise als freie Säure, als wäßrige Lösung oder gelost in einem Löse- oder Verdünnungsmittel zum Einsatz kommen. Als Löse- oder Verdünnungsmittel kommen hierbei vorzugsweise Wasser und/oder organische Lösemittel wie z. B. Alkohole (Methanol, Ethanol, Isopropanol, Cyclohexanol), Ether (Diethylether, Tetrahydrofuran, 1,4-Dioxan), Ketone (Aceton, Methylethylketon, Cyclohexanon). Ester (Methylacetat, Ethylacetat, Ethylformiat), organische Säuren (Ameisen-, Essig-, Propion-, Milch-, Brenztraubensäure) sowie aliphatische (Pentan, Hexan, Cyclohexan) und aromatische (Toluol) Kohlenwasserstoffe in Frage.

Die Umsetzung der organischen Zinksalze mit Brenztraubensäure kann jedoch ohne weiteres auch in Abwesenheit von Löse- oder Verdünnungsmitteln durchgeführt werden.

Gemäß einer bevorzugten Ausführungsform ist es im Rahmen der vorliegenden Erfindung auch möglich, Brenztraubensäure intermediär zu erzeugen, d. h. bspw. durch Umsetzung von Alkalipyruvat, wie z. B. Natrium- oder Kaliumpyruvat mit einer anorganischen Säure, wie z. B. Schwefelsäure oder Salzsäure im Temperaturbereich von - 20 bis + 90 °C, vorzugsweise - 10 bis + 60 °C.

Das Verhältnis von organischem Zinksalz zu Brenztraubensäure kann in weiten Grenzen variiert werden, doch hat es sich als besonders vorteilhaft erwiesen, die organischen Zinksalze und Brenztraubensäure in stöchiometrischen (2 : 1) oder in annähernd stöchiometrischen Verhältnissen (1,8 bis 2,2 : 1) zur Umsetzung zu bringen.

Die Durchführung des erfindungsgemäßen Verfahrens ist weitgehend unproblematisch und kann nach den üblichen Methoden und in bekannten verfahrenstechnischen Apparaturen wie in Knetern, Mischern, Schaufeltrocknern und Rührbehältern erfolgen. Auf diese Weise werden Zinkpyruvate ohne weitere Aufarbeitungsschritte in hoher Ausbeute und Reinheit erhalten.

Die erfindungsgemäßen Zinkpyruvate besitzen wertvolle biologische und medizinische Eigenschaften und finden Anwendung als Therapeutikum zur Diabetesbehandlung, Erkältungprophylaxe, Virusinhibition, Cytoprotektion als Microbicid sowie als Radikalfänger oder als Nahrungsergänzungsmittel bzw. als Supplement zur Prophylaxe und Prävention der Zinkmangelsyndrome, wie Wachstumsstörungen bei Kindern und Heranwachsenden, Gewichtsverlust, erhöhte Infektionsanfälligkeit, schlechte Wundheilung, Geschmacks- und Geruchsstörungen, verzögerte Pubertät, Appetitverminderung, Sehstörungen, Diarrhoe, Hauterkrankungen, Haarausfall und emontiale Störungen.

Die erfindungsgemäßen Zin kpyruvate zeigen überraschenderweise antivirale Eigenschaften insbesondere gegenüber Influenza A- und B-Viren, Herpes I-, II- und III-Viren sowie den Rhinoviren, antimikrobiologische Aktivitäten gegenüber bakteriellen Infektionen, wie z. B. Pseudomonas immunofluorescens, Pseudomonas aerogenes-Stämmen, Staphylokokken und Streptokokken sowie Erregern der Sinusitis, Tonsilitis und katarrhalischer Entzündungen sowohl viruzider oder bakterielle Genese, der Pyodermie und Furunkel sowie hartnäckiger, antiseptischer Wundbehandlungen. Die antimykotischen Wirkungen beziehen sich insbesondere auf Dermatomykosen, Mykosen genereller Art verursacht durch Hefen, Schimmelpilze, Dermaphyten, Candidosen und Hefen sowie der Pityriasis versiculor. Dies bezieht sich auch auf Prophylaxe der Hautund Schleimhäute, insbesondere bei der chirugischen Wundversorgung post operationem bei (auftretenden) bakteriellen Wund- und Sekundärinfektionen im dermatologischen Bereich, sofern sie durch grampositive und/oder gramnegative meklocyclin-sensible Keime hervorgerufen werden.

Die Hemmung der Influenza A- und B-Viren durch Zinkpyruvate konnte experimentell durch die Inhibition der durch Influenza A und B induzierten viralen Hämagglutination (vHA) der Erythrozyten (RBC), sowie in vitro in Zellkulturen durch die Hemmung der viralen Infektiosität nachgewiesen werden. Durch Vergleich der ermittelten Inhibitions-Konstanten der virusinduzierten Infektiosität (Plaque Reduktion, PFU = plaque forming units) in Madin-Daby-Canine-Kidney (MDCK)-Zellen, der Zytotoxizität des Zinkpyruvates, sowie der vHA auf der Ebene der IC₅₀-Konzentrationen, war es möglich, a) die Potenzierung der antiviralen Wirkung am Beispiel der IC₅₀ oder vHA und der Reduktion der Infektiosität durch Zinkpyruvat nachzuweisen; b) die zytoprotektive Wirkung von Zinkpyruvat bei Konzentrationen nachzuweisen, bei denen sowohl Pyruvat als auch Zinksalze (anorganisch wie organisch) keine biologische Wirkung, insbesondere inhibitorische Aktivitäten, zeigen, und c) der aufgrund seiher sehr niedrigen Zytotoxizität hohen therapeutischen Index des Zinkpyruvats (2 000), bzw. ein sehr hohes in-vitro-Selektivitätsverhältnis für Zinkpyruvat zu zeigen, das weder für Pyruvat noch für Zinksalze (Zink-lonen) besteht. Der gleiche therapeutische Index kann durch Administration der beiden Komponenten, wie Zn²⁺-lonen und Pyruvat, alleine oder als lockere Kombination, oder in aufeinanderfolgender Weise nicht erreicht werden. In diesem Fall sinkt der therapeutische Index sogar auf 340, wenn z. B. erst Pyruvat und dann Zn²⁺ den Zellkulturen zugesetzt werden, im umgekehrten Fall (erst Zn²⁺, dann Pyruvat) sogar auf 250. Folglich hat die Verabreichung des erfindungsgemäßen Zinkpyruvats eine synergistische Wirkung, die zu signifikanten Vorteilen gegenüber einer Verabreichung in Form der Einzelkomponenten Zink und Pyruvat führt.

Ein weiterer synergistischer Effekt des Zinkpyruvates besteht in der Reduktion von aktiven Sauerstoff- und/oder Hydroxyl-Radikalen, insbesondere in entzündlichen Prozessen. So werden fehlgeleitete lytische Reaktionen, an denen sowohl aggressive Sauerstoff- als auch Stickstoffoxid-Radikale beteiligt sind, als pathogene Mechanismen bei entzündlichen Erkrankungen hervorgerufen, durch Zinkpyruvat verhindert. Bspw. wird u. a, die zytotoxische Wirkung der Folgeprodukte dieser aggressiven Radikale durch Zinkpyruvat unterbunden und u. a. so auch Depolymerisations-Erscheinungen von Hyaluronsäure, Proteoglykanen, Kollagenfibrillen, Cytoskeletons sowie Adhesin-Proteinen und mukösen als auch membranösen Geweben supprimiert bzw. eine zeitlich begrenzte Zellprotektion bewirkt, wodurch die Infiltration aggressiver Radikale verhindert werden kann.

Zirkulierende Monozyten, die in Abwesenheit von inflammatorischen Stimulantien das Gewebe penetrieren und somit zu lokalen Makrophagen ("resident macrophages") werden, spielen nach Aktivierung z. B. durch Superoxide (Hydroperoxide), NO und O₂-Radikale sowie durch inflammatorische Ereignisse eine große Rolle in der Aktivierung von Enzymen, Wachstumsfaktoren, Zytokinen und Lymphokinen. Lymphokine werden an inflammatorischen Stellen reduziert, um Abwehrmechanismen gegen die Invasion von bakteriellen oder viralen Einflüssen (LPS, Hämagglutinin, Neuramidase, Laminine, s-ICAM) einzuleiten. Zinkpyruvat unterstützt bzw. triggert die Stimulierung der "host-defense function" durch Sekretion von Interferon-y (IFN-y) und nimmt damit an der sekretorischen Aktivität und humoraler Abwehr über die Biosynthese IL-4 und IL-10 regulativ teil. Insbesondere der regulierende und z. T. inhibitorische Einfluß von Zinkpyruvat auf die Freisetzung von HO₂^{⊖} konnte durch die experimentelle Messung der "respiratory burst activity" quantitativ unter dem Einfluß von Zinkpyruvat nachgewiesen werden.

Schließlich betrifft die Erfindung ein Verfahren zur Behandlung von Krankheiten ausgewählt aus der Gruppe bestehend aus Diabetes, Erkältungen, viralen und mikrobiellen Infektionen und Zinkmangel bzw. ein Verfahren zum Abfangen von Radikalen oder zur Zytoprotektion, wobei man dem zu behandelnden Patienten, bei dem es sich um einen Menschen oder ein Tier handeln kann, eine wirksame Menge eines erfindungsgemäßen Zinkpyruvats verabreicht.

Die Verabreichung erfolgt vorzugsweise oral. Es sind jedoch auch andere Verabreichungsformen, z.B. eine parenterale oder topische Verabreichung denkbar. Die verabreichte Menge des Zinkpyruvats kann - aufgrund der geringen Toxizität - innerhalb breiter Bereiche variiert werden. Die Behandlungsdauer hängt von der Art und Schwere der zu behandelnden Krankheit ab. Die erfindungsgemäßen Präparate können jedoch ohne gesundheitliche Schädigungen über einen Zeitraum von mehreren Wochen bis Monaten verabreicht werden.

Die Verabreichung erfolgt vorzugsweise in Form einer pharmazeutischen Zusammensetzung, die als Wirkstoff ein wasserlösliches Zinkpyruvat, insbesondere ein Zinkpyruvat wie zuvor definiert als Wirkstoff gegebenenfalls zusammen mit pharmakologisch unbedenklichen Träger-, Verdünnungs- und Hilfsstoffen enthält. Daneben kann die Zusammensetzung selbstverständlich weitere pharmazeutische Wirkstoffe, die gemeinsam mit dem Zinkpyruvat zur Behandlung einer Krankheit eingesetzt werden, enthalten. Beispielsweise sind Tagesdosen von 0,01 bis 25 mg pro kg Körpergewicht des zu behandelnden Patienten möglich, was für eine 70 kg schwere Person der Zufuhr einer Tagesgesamtmenge von 0,7 bis 1.750 mg entspricht.

Die nachfolgenden Beispiele sollen die Erfindung näher veranschaulichen.

### BEISPIELE

### A. Herstellungsbeispiele

### Beispiel A.1

Zu einer Lösung von 88 g (1 mol) reiner Brenztraubensäure (99%ig) in einem Gemisch aus 500 ml Ethylacetat und 1000 ml Eisessig werden bei 20 °C innerhalb 30 Minuten 105 g (0,48 mol) Zinkacetat Dihydrat gegeben und über 3 Stunden gerührt. Schließlich wird das Zinkpyruvat abgesaugt und mit 2 x 250 ml Ethylacetat gewaschen. Die Ausbeute an Zinkpyruvat Monohydrat beträgt 122 g (95 % d. Th.).

(C₃HO₃)₂Zn x 1 H₂O, ber.: C 27,99 %, H 3,13 %, Zn 25,39 %; gef.: C 28,09 %, H 3,28 %, Zn 25,44 %; Schmp. > 300 °C; IR (KBr) [1/cml: 644, 737, 854, 1190, 1350, 1372, 1412, 1650, 1703, 3222; ¹H-NMR (D₂O, 300 MHz): δ = 2,42 (s, 3H, CH₃-CO), 1,54 (s, 3H, CH₃ C(OH)₂); HPLC-Gehalt (Zinkpyruvat): 92,8 % = 99,8 % Zinkpyruvat Monohydrat.

### Beispiel A.2

Zu 455 g (5 mol) 98,7 %ige Brenztraubensäure werden bei 40 °C innerhalb 1 Stunde 55 g (0,25 mol) Zinkacetat Dihydrat zugegeben. Bei 40 °C wird weitere 3 Stunden gerührt. Nach Abkühlen auf 15 °C wird 1 Stunde nachgerührt. Schließlich wird das Zinkpyruvat abgesaugt, mit 2 x 100 ml Ethylacetat gewaschen und bei 50 °C und 15 mbar getrocknet. Die Ausbeute an Zinkpyruvat Monohydrat beträgt 61 g (95 % d. Th.).

### Beispiel A.3

Bei einer Temperatur von 15 bis 20 °C werden zu einer Suspension von 110 g (1 mol) Natriumpyruvat in 200 ml Ethylacetat über eine Zeit von 45 Minuten 64,3 g (0,49 mol) 70 %ige Schwefelsäure zugetropft. Nach 3 Stunden wird das ausgefallene Natriumsulfat abgesaugt und der Rückstand mit 2 x 40 ml Ethylacetat gewaschen. Zum Filtrat werden 250 g konzentrierte Essigsäure gegeben. Das Gemisch wird auf 35 °C erwärmt. Innerhalb von 30 Minuten werden 96,7 g (0,48 mol) Zinkacetat Monohydrat eingetragen. Die Nachrührzeit der Suspension beträgt 3 Stunden. Schließlich wird das Zinkpyruvat abgesaugt und mit 2 x 100 ml Ethylacetat gewaschen. Das Produkt wird bei 50 °C im Vakuumtrockenschrank bis zur Gewichtskonstanz getrocknet. Die Ausbeute an Zinkpyruvat Monohydrat beträgt 124 g (96 % d. Th.).

### Beispiel A.4

Zu einer Lösung von 45,5 g (0,5 mol) 98,7 %ige Brenztraubensäure in 200 ml Eisessig und 200 g Ethylacetat werden 20 g Wasser gegeben und bei 40 °C innerhalb 1 Stunde 38,9 g (0,25 mol) Zinkformiat eingetragen. Bei dieser Temperatur wird 3 Stunden gerührt. Nach Abkühlen auf 15 °C wird 1 Stunde nachgerührt. Schließlich wird das Zinkpyruvat abgesaugt, mit 2 x 100 ml Ethylacetat gewaschen und bei 50 °C und 15 mbar getrocknet. Die Ausbeute an Zinkpyruvat Trihydrat beträgt 71,4 g (97 % d. Th.).

(C₃H₃O₃)₂Zn x 3 H₂O, ber.: C 24,55 %, H 4,12 %, Zn 22,27 %; gef.: C 24,64 %, H 4,18 %, Zn 22,52 %; Schmp. > 300 °C; IR (KBr) [1/cm]: 644, 737, 854, 1190, 1350, 1372, 1412, 1650, 1703, 3222; ¹H-NMR (D₂O. 300 MHz): δ = 2,42 (s, 3H, CH₃-CO), 1,54 (s, 3H, CH₃-C(OH)₂); HPLC-Gehalt (Zinkpyruvat): 81,5 % = 99,8 % Zinkpyruvat Trihydrat.

### Beispiel A.5 (Vergleich)

Nacharbeitung der Herstellungsvorschrift des Zinkoxydsalzes der brenzlichen Traubensäure nach J.J. Berzelius, Annalen der Physik und Chemie (1835) 36, 20.

34,2 g (0,1 mol) Zinkhydroxidcarbonat Hydrat (Atdrich 36,720-6) werden in 105,6 g (0,6 mol) 50 %iger wäßriger Brenztraubensäure gelöst. Nach beendeter Kohlendioxidentwicklung wird noch 2 Stunden gerührt. Schließlich wird der entstandene weiße Niederschlag abgesaugt, mit 50 ml Wasser und 50 ml Methanol gewaschen und bei 50 °C und 15 mbar getrocknet. Die Analysen charakterisierten den Niederschlag eindeutig als Zinkparapyruvat. Die Ausbeute an Zinkparapyruvat Trihydrat beträgt 79,1 g (80 % d. Th.).

(C₆H₆O₆)Zn x 3 H₂O, ber.: C 24,55 %, H 4,12 %, Zn 22,27 %; gef.: C 24,64 %, H 4,18 %, Zn 22,52 %; Schmp. > 300 °C; IR (KBr) [1/cm]: 642, 669, 798, 1075, 1113, 1140, 1205, 1228, 1342, 1396, 1474, 1609, 1677, 1713, 3408; ¹H-NMR (D₆-DMSO, 300 MHz): δ = 1,27 (s, 3H, CH₃-CO), 2,99 (d. 1H, CH₂), 3,25 (d, 1H, CH₂); HPLC-Gehalt (Zinkparapyruvat): 81,5 % = 99,8 % Zinkparapyruvat Trihydrat.

### Beispiel A.6 (Vergleich)

6,5 g (0,1 mol) metallisches Zinkpulver werden in 35,2 g (0,2 mol) 50 %iger wäßriger Brenztraubensäure gelöst. Nach beendeter Wasserstoffentwicklung wird noch 2 Stunden gerührt. Schließlich wird der entstandene weiße Niederschlag abgesaugt, mit 50 ml Wasser und 50 ml Methanol gewaschen und bei 50 °C und 15 mbar getrocknet. Die Analysen charakterisierten den Niederschlag eindeutig als Zinkparapyruvat. Die Ausbeute an Zinkparapyruvat Tetrahydrat beträgt 24,0 g (77 % d. Th.).

### B. Anwendungsbeispiele

### Beispiel B.1

### Assays zur Makrophagen-Aktivierung und Zytokine

Es wurde ein modifizierter Assay nach Barbior et al. (B.M.Barbior, R.S.Kipnes, J.T.Cumutte,J.Clin.Invest., 52, 741, 1973) durchgeführt. Dabei wurden 5 x 10⁵ Makrophagen pro Titer und "well" in Gegenwart von 10 % fötalem Kälberserum bei steigenden Konzentrationen von Zinkpyruvat (0-0,1-1·10³ mM) eingesetzt. Die vorgelegte Zellzahl an Makrophagen entspricht bzw. gewährleistet, daß die Freisetzung von HO₂^{⊖} genau proportional der Konzentration der Zellzahl ist. Der Leerwert (blank, nur Puffer), sowie eine negative Kontrolle und eine positive Kontrolle (verum, Zinkpyruvat) wurden in der Art und Weise durchgeführt, daß auch eine Gewährleistung für die Spezifität der Superoxid-Anionen gegeben ist. So wurden die "waffe" mit 15 µg Superoxid-Dismutase bei einer Zellzahl von 3,0 x 10⁵ versetzt, um als "negative" Kontrolle zu fungieren.

Die Makrophagen wurden entweder mit Phorbol-Myristinsäureester (20 µg/ml) oder Zymosan (100/µg/ml) aktiviert. Die so stimulierten Makrophagen wurden als negative Kontrolle, "blank" oder mit der entsprechenden Konzentration von Zinkpyruvat (verum) vorbehandelt (Ermittlung der Zytoprotektion) oder direkt dem Assay zugesetzt (Bestimmung der Inhibition), anschließend mit 0,02 M NaH₂PO₄-Puffer (pH 6,5, 20°C) gewaschen, und anschließend mit 0,5 ml Reaktionsgemisch/Hanks' Lösung (Phenolrot-frei), 80 µM Ferri-Cytochrom C (Sigma Typ IV) und 5 mM NaN₃, das als Cytochrome-Oxidase-inhibitor fungiert, sowie mit Stimulans (≈ 50 - 70 µl) bei 37 ° C für 20 min inkubiert. Die Cytochrom C-Reduktion wurde durch die Änderung der Extinktion bei 550 nm gemessen. Über die Absorptionsdifferenz bei 550 nm in Abwesenheit oder Anwesenheit von Superoxid-Dismutase unter Verwendung des experimentell ermittelten (Arbeits-)Extinktions-Koeffizienten von 18,95/mM/cm (reduziert oder oxidiert), wurde die Konzentration an Superoxid-Anionen bestimmt. Die biochemischen Aktivitäten der Hemmung der Sauerstoff-Radikalbildung wurden entsprechend der Michaelis-Menten-Kinetik gemessen bzw. evaluiert.

Die Hemmung der Freisetzung der Superoxid-Radikale (Anionen) in Gegenwart von Zinkpyruvat ergab eine Hemmkonstante von Kᵢ = 550 ± 25 mM bei einer Assoziationskonstante von Zinkpyruvat an die Makrophagen unter diesen in-vitro-Bedingungen von 150 nM. Bei Vorbeha: dlung der Makrophagen mit 1,0 mM Zinkpyruvat für einen Zeitraum von 10 min bei 37 °C und anschließende Stimulierung mit Phorbolmyristinsäureester unter gleichen Assaybedingungen wurden Inhibitions-Konstanten von Kᵢ₍₁₎ = 150 ± 25 mM bzw. von Kᵢ₍₁₎ = 670 ± 55 mM gefunden. Die beiden verschiedenen Hemmkonstanten erklären sich durch die allosterische Inhibition der vorbehandelten Makrophagen in Bezug auf die Freisetzung von HO₂^{⊖} durch bereits gehemmte Makrophagen, welche dem Assay nicht mehr zur Verfügung stehen bzw. refraktär (allosterische Hemmung) geworden sind. Die zusätzlich durch Gleichgewichts-Dialyse ermittelte Bindungskonstante von nur 150 nM Zinkpyruvat an die Makrophagen bei einer Zellzahl von 10⁵ belegt außerdem den ungewöhnlichen allosterischen Hemmverlauf in Gegenwart von Zinkpyruvat. Trotz Erhöhung der Konzentration des Stimulans, allerdings unter gesättigten Bedingungen (Zinkpyruvat-/Makrophage), änderten sich weder die Bindungs-Konstanten für Zinkpyruvat und die der Makrophagen, noch die Hemmkonstanten Kᵢ, es wurden aber pro Zeiteinheit mehr Sauerstoff-Radikale inaktiviert. Dies bewirkt, daß sich in Gegenwart von Zinkpyruvat die "turnover-number" der Makrophagen zur Vernichtung der aktiven und aggressiven Sauerstoff-Radikale von ≅350 auf ≅ 7 100 erhöht.

### Beispiel B.2

### Assay zur Inhibierung der Sekretion von NO

Der Assay für die Beeinflussung der Sekretion von Stickoxid durch Zinkpyruvat wurde nach der GRIESS Reaktion ermittelt (A.H.Ding, C.F.Nathan & D.J.Stuehr, J.lmmunol., 141, 2407, 1988). Das modifizierte GRIESS Reagenz setzte sich folgendermaßen zusammen: 1 ml 0,5 % (g/g) Naphthalinethylendiamin • H₃PO₄, 1 ml 0,5 % (g/g) Sulfanilsäureamid in 1 % (g/g) H₃PO₄. Um den NO-Ausbruch ("burst") für Makrophagen zu aktivieren, wurden die Makrophagen (Zelizahl: 2,0 x 10⁵) in 500 µl GRIESS-Reagenz durch Zugabe von 75 Einheiten/ml von IFN-y für 2 Std. bei 37 °C inkubiert. Nach Zugabe von 20µg/ml Lipopolysaccharid (LPS, E.coli, MRE 600) wurde die NO-Bestimmung ausgelöst bzw. gestartet. Inkubiert wurde für 12 Std. bei 37 °C unter N₂-Belüftung. Als negative bzw. positive Kontrolle bei der NO-Biosynthese wurden 200 µg/ml N⁶-Monoethylarginin zusammen mit LPS zugesetzt. Der Überstand wurde durch Zentrifugation von den Zellen abgetrennt, das entsprechende Volumen mit dem Griess-Reagenz versetzt, und anschließend nach 5 min. bei 525 nm die Extinktion gemessen (Glasküvette, 1 cm Schichtdicke, 25 °C).

Die ermittelten Hemmkonstanten für Zinkpyruvat beliefen sich auf Kᵢ = 600 ± 50 mM, bei Vorbehandlung der Makrophagen mit Zinkpyruvat auf Kᵢ = 230 ± 60 mM, allerdings mit nur einer Hemmkonstanten nach Michaelis-Menten. D. h. durch die konzentrationsabhängige Hemmung der aktiven, aggressiven und zykotischen Sauerstoffradikale und Hydroperoxide (je nach pH) sowie der zytosolischen Aktivierung der Zellen durch Zinkpyruvat können sowohl die humoralen als auch die zellulären Aktivierungskomponenten einschließlich des MHC III-Komplexes am Makrophagen Mannosyl-Rezeptor moduliert werden. Diese Modulierung schließt außerdem die IL-1,IL-6 sowie die TNF-α-Faktoren ein. Diese Elemente besetzen insbesondere die pro-inflammatorischen Stellen bei entsprechender Reduzierung der pro-inflammatorischen Zytokin-Sekretion durch Zinkpyruvat. Dabei werden die Makrophagen in Gegenwart von Zinkpyruvat und im Gegensatz zu IFN-γ, IL-10, IL-4 und IL-13 zwar im aktivierten Zustand gehalten, aber so, daß sie die notwendigen protektiven Zytokine, wie IL-1, IL-6 und TNF-α stimulieren und damit einen Schutz gegen LPS, bakterielle Produkte (Fieber, Ödem, Freisetzung von Prostaglandinen und ev. Leukotrienen, SLS-Produkten) und auch gegen virale Produkte gewährleisten (Erhöhung der Autoimmun-Aktivitäten) können. Somit leistet das Zinkpyruvat durch zelluläre Induktion einen Beitrag zur Reduzierung der inflammatorischen Ereignisse, indem sie die Antagonisten der pro-inflammatorischen Zytokine stimulieren.

### Beispiel B.3

### In-vitro-Hemmung der viralen Hämagglutination (vHAI) und der Infektiosität durch Reduktion der Plaques in Gegenwart von Zinkpyruvat

Wie bereits erwähnt, wurden die antiviralen Eigenschaften der Zinkpyruvate an epithelialen MDCK-Zellen für die Influenza-Stämme A/Chile/1/83/ oder Influenza B/Singapore/27/79/ getestet. Die Assay-Bedingungen entsprachen denen von K.Tabita, A. Sugiura, C.Enomoto, M. Furuyama, Med. Micobiol.Immunol., 165, 9-14, 1975. Die infizierten MDCK Zellen wurden 30 min mit Zinkpyruvat behandelt und verglichen mit Zellkulturen, welche nicht mit Influenza-Virus infiziert worden waren. Eine qualitative und quantitative Kontrolle der fortschreitenden Infektion erfolgte zusätzlich durch Bestimmung der viralen Proteine sowie der Zahl der infektiösen Partikel im Zellkultur-Überstand.

Die Bestimmung der Zell-Zytotoxizität wurde entsprechend unter identischen Kulturbedingungen mit den gleichen, aber nicht infizierten MDCK-Zellen durchgeführt. Die Validität der erhaltenen Ergebnisse wurde auf Basis der 95 % Vertrauensgrenze unter Anwendung der "t"-Test-Werte für die entsprechenden Konfidenzbereiche ermittelt.

Die Hemmung der durch Influenza induzierten Hämagglutination (vHAI) von Erythrozyten (RBC) wurde entsprechend der Vorschrift von G.N.Rogers, T.Pritchett, J.L.Lane, J.C.Paulsen, Virology, 131, 394-408, 1983, durchgeführt. Die Ergebnisse sind in Tabelle 1 zusammengefaßt.

Bemerkenswert und überraschend ist die geringe Zytotoxizität von Zinkpyruvat. Die Lysis der RBC-Zellen beginnt bei 1 000 µg/ml, also erheblich höher als für die im vHAI ermittelten Hemmkonstanten, die in der Größenordnung von 50 µg/ml liegen. Die LD₅₀ Konzentration für die MDCK-Zellen ergibt im Druchschnitt Werte von 800 bis 1 000 µg/ml für Zinkpyruvat. D. h. alle gefundenen Werte liegen weit über denen der biochemisch aktiven Inhibitor-Konzentrationen, so daß die therapeutischen Konzentrationen an Zinkpyruvat in keiner Weise einen zytotoxischen Einfluß auf die Zellen in-vitro ausüben, und weshalb die gemessene Hemmung der einz Inen Parameter auf Grund einer spezifischen Wechselwirkung mit dem Zellapparat oder dem Virus zurückzuführen ist und nicht auf die Lysis der MDCK-Zellen.

**Tabelle 1**

| Hämagglutination-Inhibition (HAI), Plaque Reduktions-Assay von Zn-Pyruvat | | | | | |
|---|---|---|---|---|---|
| Nr. | Inhibitor | HAI, µm IC₅₀ | Potenz HAI | Plaque µM | Reduktion^{a} % |
| 1 | Zn(Ac)₂ | 150,0 | 6,0 | 1700 | 15 |
| 2 | Zn(Py)₂ | 25,3 | 19,9 | 90,5 | 67 |
| 3 | ZnCl₂ | 100,0 | 5,0 | 1200 | 20 |
| 4 | ZnO•H₂O | ≈ 200,0 | 4,5 | 1700 | 15 |

| | | | | | |
|---|---|---|---|---|---|
| a) Die Werte stellen die prozentuale Reduktion der Plaque-Zahl pro Volumeneinheit (well) dar, die durch die virale Lysis der infizierten Zellen hervorgerufen werden. | | | | | |

## Patentansprüche

1. Wasserlösliche Zinkpyruvate bzw. deren Hydrate der Formel 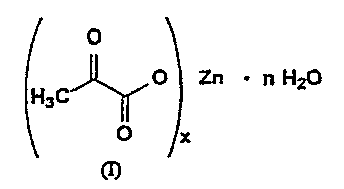 mit x= 1,8 bis 2,2 und n=0 bis 5.

2. Zinkpyruvate nach Anspruch 1,
**dadurch gekennzeichnet,**
**daß** x = 2 und n = 0 bis 3 bedeuten.

3. Zinkpyruvate nach Anspruch 1 oder 2,
**dadurch gekennzeichnet,**
**daß** das Pyruvat-Anion als 2,2-Dihydroxypropionat-Anion vorliegt.

4. Zinkpyruvate nach einem der Ansprüche 1 bis 3,
**dadurch gekennzeichnet,**
**daß** sie in fester mikrokristalliner Form vorliegen.

5. Verfahren zur Herstellung der Zinkpyruvaten nach einem der Ansprüche 1 bis 4,
**dadurch gekennzeichnet,**
**daß** man Zinksalze von organischen Säuren oder aciden organischen Keto- oder Hydroxyverbindungen mit Brenztraubensäure im Temperaturbereich von -20 bis +90°C, ggf. in Gegenwart eines Löse- oder Verdünnungsmittels umsetzt.

6. Verfahren nach Anspruch 5,
**dadurch gekennzeichnet,**
**daß** man die Umsetzung in einem Temperaturbereich von 10 bis 50°C durchführt.

7. Verfahren nach Anspruch 5,
**dadurch gekennzeichnet,**
**daß** man als organische Säure eine (Amino-, Keto- und/oder Hydroxy-)-Carbonsäure verwendet.

8. Verfahren nach einem der Ansprüche 5 bis 6,
**dadurch gekennzeichnet,**
**daß** man als organische Säure oder acide organische Keto- oder Hydroxyverbindung eine Säure ausgewählt aus der Gruppe Ameisensäure, Essigsäure, Propionsäure, Buttersäure, Milchsäure, Ascorbinsäure, Citronensäure, Weinsäure, Bernsteinsäure, Maleinsäure, Fumarsäure, Äpfelsäure, Asparaginsäure, Benzoesäure, Gluconsäure, Isovaleriansäure, Ölsäure, Glycin oder Lysin einsetzt.

9. Verfahren nach einem der Ansprüche 5 bis 8,
**dadurch gekennzeichnet,**
**daß** die Zinksalze der organischen Säuren oder aciden organischen Keto- oder Hydroxyverbindungen in wasserfreier Form, als Hydrate oder als feuchte Produkte verwendet werden.

10. Verfahren nach einem der Ansrüche 5 bis 9,
**dadurch gekennzeichnet,**
**daß** die Brenztraubensäure als wasserfreie Säure, als wässrige Lösung oder gelöst in einem Löse- oder Verdünnungsmittel eingesetzt wird.

11. Verfahren nach einem der Ansprüche 5 bis 10,
**dadurch gekennzeichnet,**
**daß** man als Löse- oder Verdünnungsmittel ein organisches Lösemittel und/oder Wasser verwendet.

12. Verfahren nach Anspruch 11,
**dadurch gekennzeichnet,**
**daß** man als organische Lösemittel Alkohole, Ether, Ketone, Ester, organisches Säuren sowie aliphatische und aromatische Kohlenwasserstoffe einsetzt.

13. Verfahren nach einem der Ansprüche 5 bis 12,
**dadurch gekennzeichnet,**
**daß** die Brenztraubensäure intermediär gebildet wird, bspw. durch Umsetzung von Alkalipyruvat mit einer anorganischen Säure wie z.B. Schwefel- oder Salzsäure im Temperaturbereich von -20 bis + 90°C, vorzugsweise -10 bis + 60°C.

14. Pharmazeutische Zusammensetzung,
**dadurch gekennzeichnet,**
**daß** sie als Wirkstoff ein wasserlösliches Zinkpyruvat, insbesondere ein Zinkpyruvat nach einem der Ansprüche 1 bis 4 gegebenenfalls zusammen mit pharmazeutisch unbedenklichen Träger-, Verdünnungs- und Hilfsstoffen enthält.

15. Verwendung der Zinkpyruvate nach einem der Ansprüche 1 bis 4 oder der Zusammensetzung nach Anspruch 14 zur Herstellung eines Mittels, insbesondere eines Therapeutikums zur Diabetesbehandlung, Erkältungsprophylaxe, Virusinhibition, Cytoprotektion, eines Microbicids und eines Radikalfängers.

16. Verwendung der Zinkpyruvate nach einem der Ansprüche 1 bis 4 oder der Zusammensetzung nach Anspruch 14 zur Herstellung eines Nahrungsergänzungsmittels bzw. Supplements zur Prophylaxe und Prävention der Zinkmangelsymptome wie Wachstumsstörungen bei Kindern und Heranwachsenden, Gewichtsverlust, erhöhte Infektionsanfälligkeit, schlechte Wundheilung, Geschmacks- und Geruchsstörungen, verzögerte Pubertät, Appetitverminderung, Sehstörungen, Diarrhoe, Hauterkrankungen, Haarausfall und emontiale Störungen.

## Claims

1. A water-soluble zinc pyruvate and hydrate thereof of the formula I 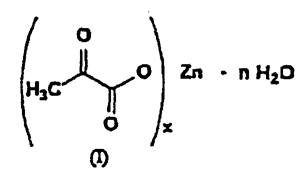 where x = 1.8 to 2.2 and n = 0 to 5.

2. A zinc pyruvate as claimed in claim 1, wherein x = 2 and n = 0 to 3.

3. A zinc pyruvate as claimed in claim 1 or 2, wherein the pyruvate anion is present as 2,2-dihydroxypropionate anion.

4. A zinc pyruvate as claimed in one of claims 1 to 3, wherein it is present in solid microcrystalline form.

5. A process for preparing zinc pyruvates as claimed in one of claims 1 to 4, wherein zinc salts of organic acids or acidic arganic keto or hydroxy compounds are reacted with pruvic acid in the temperature range from -20 to +90°C, in the presence or absence of a solvent or diluent.

6. The process as claimed in claim 5, wherein the reaction is carried out in a temperature range from 10 to 50°C.

7. The process as claimed in claim 5, wherein the organic acid used is an (amino-, keto- and/or hydroxy-)carboxylic acid.

8. The process as claimed in one of claims 5 to 6, wherein the organic acid or acidic organic keto compound or hydroxyl compound used is an acid selected from the group consisting of formic acid, acetic acid, propionic acid, butyric acid, lactic acid, ascorbic acid, citric acid, tartaric acid, succinic acid, maleic acid, fumaric acid, malic acid, aspartic acid, benzoic acid, gluconic acid, isovaleric acid, oleic acid, glycine or lysine.

9. The process as claimed in one of claims 5 to 8, wherein the zinc salts of the organic acids or acidic organic keto compounds or hydroxyl compounds are used in anhydrous form, as hydrates or as moist products.

10. The process as claimed in one of claims 5 to 9, wherein the pyruvic acid is used as anhydrous acid, as aqueous solution or dissolved in a solvent or diluent.

11. The process as claimed in one of claims 5 to 10, wherein the solvent or diluent used is an organic solvent and/or water.

12. The process as claimed in claim 11, wherein alcohols, ethers, ketones, esters, organic acids and aliphatic and aromatic hydrocarbons are used as organic solvent.

13. The process as claimed in one of claims 5 to 12, wherein the pyruvic acid is formed as an intermediate, for example by reacting alkali metal pyruvate with an inorganic acid, for example sulfuric acid or hydrochloric acid, in the temperature range from -20 to +90°C, preferably from -10 to +60°C.

14. A pharmaceutical composition which comprises as active compound a water-soluble zinc pyruvate, in particular a zinc pyruvate as claimed in one of claims 1 to 4 with or without pharmaceutically compatible excipients, diluents and aids.

15. The use of the zinc pyruvate as claimed in one of claims 1 to 4 or the composition as claimed in claim 14 for producing a preparation, in particular a therapeutic for treating diabetes, cold prophylaxis, virus inhibition, cytoprotection, a microbicide and a free-radical scavenger.

16. The use of the zinc pyruvate as claimed in one of claims 1 to 4 or the composition as claimed in claim 14 for producing a food supplement or supplement for the prophylaxis and prevention of zinc deficiency symptoms such as growth disorders in children and adolescents, weight loss, increased susceptibility to infection, poor wound healing, taste and odor disorders, delayed puberty, loss of appetite, vision disorders, diarrhea, skin disorders, hair loss and emotional disorders.

## Revendications

1. Pyruvates de zinc hydrosolubles ou ses hydrates de formule: 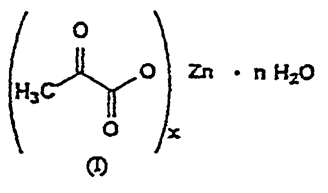 avec x de 1,8 à 2,2 et n de 0 à 5.

2. Pyruvates de zinc selon la revendication 1,
**caractérisés par le fait**
**que** x = 2 et n = de 0 à 3.

3. Pyruvates de zinc selon les revendications 1 ou 2,
**caractérisés par le fait**
**que** l'anion de pyruvate est un anion 2,2-dihydroxypropionate.

4. Pyruvates de zinc selon les revendications 1 à 3,
**caractérisés par le fait**
**qu'**ils existent sous forme micro-cristalline solide.

5. Procédé de fabrication de pyruvates de zinc selon une des revendications 1 à 4,
**caractérisé par le fait**
**que** l'on fait réagir des sels de zinc issus d'acides organiques ou de composés acides organiques cétoniques ou hydroxyliques avec de l'acide pyruvique à des températures de -20° à +90°C, éventuellement en présence d'un solvant ou d'un agent de dilution.

6. Procédé selon la revendication 5,
**caractérisée par le fait**
**que** la réaction s'effectue de 10 à 50°C.

7. Procédé selon la revendication 5 à 6,
**caractérisé par le fait**
**que** l'on peut utiliser comme acide organique un (amino-, céto- et/ou hydroxy-)acide carboxylique.

8. Procédé selon les revendications 5 à 6,
**caractérisé par le fait**
**que** l'on introduit comme acide organique ou comme composé acide organique cétonique ou hydroxylique un acide sélectionné dans le groupe acide formique, acide acétique, acide propionique, acide butyrique, acide lactique, acide ascorbique, acide citrique, acide tartrique, acide succinique, acide de maléine, acide fumarique, acide malique, acide asperginique, acide benzoïque, acide gluconique, acide isovalérianique, acide oléique, glycine ou lysine.

9. Procédé d'après une des revendications 5 à 8,
**caractérisé par le fait**
**que** les sels de zinc des acides organiques ou des composés acides organiques cétoniques ou hydroxylique sont utilisés sous forme anhydre, comme hydrates ou comme produits humides.

10. Procédé d'après une des revendications 5 à 9,
**caractérisé par le fait**
**que** l'acide pyruvique utilisé est anhydre, en solution aqueuse ou dissout dans un solvant ou un diluant.

11. Procédé selon une des revendications 5 à 10.
**caractérisé par le fait**
**que** l'on utilise un solvant organique ou de l'eau comme solvant ou diluant.

12. Procédé selon la revendication 11,
**caractérisé par le fait**
**que** l'on utilise comme solvants organiques des alcools, des éthers, des cétones, des esters, des acides organiques ainsi que des hydrocarbures aromatiques et aliphatiques.

13. Procédé selon une des revendications 5 à 12,
**caractérisé par le fait**
**que** l'acide pyruvique est formé de façon intermédiaire en faisant, par exemple, réagir du pyruvate alcalin avec un acide inorganique tel l'acide sulfurique ou l'acide chlorhydrique de -20 à +90°C, de préférence de -10 à +60°C.

14. Composition pharmaceutique,
**caractérisée par le fait**
**qu'**elle contient comme principe actif du pyruvate de zinc hydrosoluble, en particulier un pyruvate de zinc selon une des revendications 1 à 4, le cas échéant avec des supports, des diluants et des excipients ne présentant pas de risques pharmaceutiques.

15. Utilisation des pyruvates de zinc selon les revendications 1 à 4 ou du composé selon la revendication 14 pour fabriquer un médicament, destiné en particulier au traitement du diabète, à la prophylaxie du refroidissement, à l'inhibition virale, à la cytoprotection, et également à la fabrication d'un microbicide et d'un piégeur de radicaux libres.

16. Utilisation des pyruvate de zinc selon une des revendications 1 à 4 ou du composé selon la revendication 14 pour la fabrication d'un supplément alimentaire destiné, par exemple, à la prophylaxie et à la prévention des symptômes dus à la carence en zinc tels les troubles de croissance chez l'enfant et l'adolescent, la perte de poids, l'augmentation de la fragilité aux infections, la mauvaise cicatrisation, les troubles du goût et de l'odorat, le retard pubertaire, la diminution de l'appétit, les problèmes de vue, la diarrhée, les maladies de la peau, la chute des cheveux et les troubles émotionnels.
